# EUROPÄISCHE PATENTSCHRIFT

(11) **EP 2 582 648 B1**
(45) Veröffentlichungstag und Bekanntmachung des Hinweises auf die Patenterteilung: **30.04.2014**
(21) Anmeldenummer: 11726396.2
(22) Anmeldetag: 14.06.2011
(51) Int. Cl.: C07C 2/10, C07C 2/12, C07C 11/02

(54) **VERFAHREN ZUR CO-OLIGOMERISIERUNG VON OLEFINEN**
PROCESS FOR CO-OLIGOMERIZATION OF OLEFINS
PROCÉDÉ DE CO-OLIGOMÉRISATION D'OLÉFINES

(30) Priorität: 15.06.2010 EP 10166012
(43) Veröffentlichungstag der Anmeldung: 24.04.2013
(73) Patentinhaber: BASF SE, 67056 Ludwigshafen (DE)
(72) Erfinder: ROHDE, Wolfgang, 67346 Speyer (DE); MIAO, Qiang, 67549 Worms (DE); BITTERLICH, Stefan, 67246 Dirmstein (DE); AVERLANT, Gauthier Luc Maurice, 60323 Frankfurt (DE); WAGNER, Hans-Günter, 67271 Neuleiningen (DE); RÖSSLER-FEIGEL, Beatrice, 67256 Weisenheim am Sand (DE)
(86) Internationale Anmeldenummer: PCT/EP2011/059770
(87) Internationale Veröffentlichungsnummer: WO 2011/157674

(56) Entgegenhaltungen:
- WO-A1-2007/141288
- GB-A- 1 124 765
- R.G. DA ROSA ET AL: "Oligomerization and co-oligomerization of [alpha]-olefins catalyzed by nickel(II)/alkylaluminum systems", JOURNAL OF MOLECULAR CATALYSIS A: CHEMICAL, Bd. 120, Nr. 1-3, 1. Juni 1997 (1997-06-01), Seiten 55-62, XP55005862, ISSN: 1381-1169, DOI: 10.1016/S1381-1169(96)00438-4
- Y. V. KISSIN: "Co-oligomerization of ethylene and higher linear alpha olefins. I. Co-oligomerization with the sulfonated nickel ylide-based catalytic system", JOURNAL OF POLYMER SCIENCE PART A: POLYMER CHEMISTRY, Bd. 27, Nr. 2, 30. Januar 1989 (1989-01-30), Seiten 605-621, XP55005907, ISSN: 0887-624X, DOI: 10.1002/pola.1989.080270220

## Beschreibung

Die Erfindung betrifft ein Verfahren zur Co-Oligomerisierung von Olefinen, bei dem man ein Olefin-Einsatzmaterial, das Olefine mit n Kohlenstoffatomen und Olefine mit 2n Kohlenstoffatomen umfasst, an einem Olefin-Oligomerisierungskatalysator umsetzt.

Kurzkettige Olefine sind im großtechnischen Maßstab erhältlich. So fällt z. B. bei der Aufarbeitung von Erdöl durch Steamcracken oder Fluidized Catalyst Cracking (FCC) ein als C₄-Schnitt bezeichnetes Kohlenwasserstoffgemisch mit einem hohen Gesamtolefingehalt an, wobei es sich im Wesentlichen um Olefine mit 4 Kohlenstoffatomen handelt.

Höhere Olefine werden häufig durch Oligomerisation niederer, monomerer Olefine gewonnen. Die Oligomerisation erfolgt an homogenen oder heterogenen Katalysatoren. Solche Katalysatoren lassen sich in zwei große Klassen unterteilen, den aciden Katalysatoren und den koordinativen Katalysatoren. Zu der ersteren Klasse gehören Zeolithe in der H-Form, zur letzteren gehören z. B. Nickeloxid-basierte Katalysatoren. Bei unsymmetrischen Olefinmonomeren hängt der Aufbau des Produkts wesentlich davon ab, an welchem der beiden unterschiedlichen C-Atome der Doppelbindung des Monomeren, der Kettenaufbau erfolgt. So entstehen mehr oder weniger stark verzweigte olefinische Oligomere mit mehr oder weniger stark substituierten Doppelbindungen. Da die olefinischen Oligomere ihrerseits mit sich selbst bzw. mit weiterem Monomer reagieren können und außerdem eine Verschiebung der Doppelbindungen eintreten kann, ist die Oligomerisation von Olefin-Monomeren hoch komplex und in der Regel nicht vollständig beschreibbar. Eine Übersicht findet sich in S. Albrecht et al., Chemie Ingenieur Technik, 77, 695 (2005).

Man erhält daher stets mehrere Produkte, die sich im Oligomerisierungsgrad, also in der Kettenlänge bzw. C-Zahl, unterscheiden. Die verschiedenen Oligomere werden in der Regel nach ihrer C-Zahl aufgetrennt und verschiedenen Anwendungen zugeführt. Die Oligomere gleicher C-Zahl sind ihrerseits komplexe Gemische verschiedener Isomere.

Will man die Selektivität zu höheren als dimeren Oligomeren erhöhen, so kann man anstelle von reinem Monomer auch Mischungen aus Monomeren und Dimeren als Einsatzmaterial für die Oligomerisation einsetzen. Dabei findet neben der Co-Dimerisierung zwischen Dimer und Monomer meist auch eine Homo-Dimerisierung des Monomers statt. Üblicherweise wird in einer ersten Reaktionseinheit reines Monomer oligomerisiert und in einer oder mehreren nachfolgenden Reaktionseinheiten werden Monomer und Oligomere miteinander zur Reaktion gebracht.

Die WO 01/83407 offenbart ein Verfahren zur Oligomerisierung von Alkenen mit 3 bis 6 Kohlenstoffatomen, bei dem man einen Zulauf, der (a) Alkene mit x Kohlenstoffatomen und (b) gegebenenfalls Alkene mit y Kohlenstoffatomen enthält (wobei x und y verschieden sind) mit einem MFS-Zeolith-Katalysator in Kontakt bringt. Die Bedingungen sind so gewählt, dass man selektiv ein oligomeres Produkt mit Hauptanteilen bestimmter Oligomere erhält.

Die WO 2007/040812 beschreibt ein Verfahren zum Umwandeln niederer Olefine in höhere Olefine, bei dem man einen Zulauf, der ein C₃-C₅-Olefinmonomer und ein Dimer des Monomers enthält, mit einem Zeolith-Oligomerisierungskatalysator in Kontakt bringt und ein Trimer des Olefinmonomers erhält.

Die WO 2007/141288 beschreibt ein Verfahren zur Co-Dimerisierung von Olefinen, bei dem man ein erstes Olefin-Einsatzmaterial, das im Wesentlichen aus Cₙ-Olefinen besteht, und ein zweites Olefin-Einsatzmaterial bereitstellt, das im Wesentlichen aus Cₘ-Olefinen besteht, wobei n und m unabhängig voneinander für zwei voneinander verschiedene ganze Zahlen von 2 bis 12 stehen, und das erste und das zweite Olefin-Einsatzmaterial an einem heterogenen Olefin-Oligomerisierungskatalysator, insbesondere einem Olefin-Oligomerisierungskatalysator auf Basis eines Schicht- und/oder Gerüstsilicats, umsetzt.

Die EP-A 1739069 beschreibt die Herstellung einer Dieselfraktion, wobei man eine C₂-C₁₂-olefinische Kohlenwasserstofffraktion oligomerisiert, das erhaltene Gemisch auftrennt in eine Leichtfraktion mit nicht umgesetzten C₄- und/oder C₅-olefinischen Kohlenwasserstoffen, eine Mittelfraktion und eine Schwerfraktion, und die Mittelfraktion mit der Leichtfraktion in einem Gewichtsverhältnis von 60:40-80:20 oligomerisiert.

Werden zur Oligomerisierung Olefin-Isomerengemische eingesetzt, so reagieren in der Regel bevorzugt zunächst die reaktiveren Isomere. Es kann zu einer starken Verarmung des Reaktionsgemischs an reaktiven Isomeren kommen, bevor auch die weniger reaktiven Olefine an der Oligomerisierung teilnehmen. Da die Oligomerisierungsreaktion meist unter Teilumsatz durchgeführt wird, können sich im Extremfall nicht umgesetzte weniger reaktive Olefine vollständig im Reaktionsaustrag wieder finden.

Häufig werden die erhaltenen olefinischen Oligomere anschließend durch Hydroformylierung in die um jeweils ein C-Atom längeren Alkohole (Oxo-Alkohole) umgewandelt, die dann ihrerseits wichtige Grundprodukte für Weichmacher und Tenside darstellen. Die Hydroformylierbarkeit eines Olefins (d.h. die mit üblichen Methoden bestimmte Reaktionsgeschwindigkeit der Hydroformylierung unter definierten Bedingungen) hängt von dem Verzweigungsgrad des zu hydroformylierenden Olefins und vom Substitutionsgrad der olefinischen Doppelbindungen ab. Nach B. Heil et al. (Chem. Ber., 102, 2238-2240 (1969)) fällt die Hydroformylierbarkeit von Olefinen in folgender Reihe: lineare α-Olefine > lineare innenständige -Olefine> verzweigte Olefine, insbesondere substituierte Doppelbindungen.

Der Erfindung liegt die Aufgabe zugrunde, ein Verfahren zur Co-Oligomerisierung von Olefinen anzugeben, bei dem man ein Olefin-Einsatzmaterial, das Olefine mit n Kohlenstoffatomen und Olefine mit 2n Kohlenstoffatomen umfasst, an einem Olefin-Oligomerisierungskatalysator zu einem Umsetzungsprodukt umsetzt und bei dem sowohl das Co-Oligomer mit 3n Kohlenstoffatomen als auch das vom Umsetzungsprodukt abgetrennte Olefin mit 2n Kohlenstoffatomen eine möglichst hohe Hydroformylierbarkeit aufweisen.

Die Aufgabe wird gelöst durch ein Verfahren zur Co-Oligomerisierung von Olefinen, bei dem man ein Olefin-Einsatzmaterial, das Olefine mit n Kohlenstoffatomen und Olefine mit 2n Kohlenstoffatomen umfasst, an einem Olefin-Oligomerisierungskatalysator zu einem Umsetzungsprodukt umsetzt, wobei man das Verfahren unter solchen Bedingungen durchführt, dass der Umsatz an Olefinen mit 2n Kohlenstoffatomen nicht negativ und kleiner als 5 % ist und der Umsatz der Olefine mit in Kohlenstoffatomen im Bereich von 10 bis 50 % liegt.

Der Koeffizient n steht für eine ganze Zahl von 3 bis 10, bevorzugt 4 bis 6.

Bei der Co-Oligomerisierung werden gleichzeitig (a) Olefine mit 2n Kohlenstoffatomen zu Olefinen mit 3n und/oder mehr Kohlenstoffatomen umgesetzt und (b) Olefine mit n Kohlenstoffatomen zu Olefinen mit 2n Kohlenstoffatomen dimerisiert. Der Umsatz (bzw. Netto-Umsatz) an Olefinen mit 2n Kohlenstoffatomen ist die Differenz der gemäß (a) verbrauchten Menge an Olefinen mit 2n Kohlenstoffatomen und der gemäß (b) gebildeten Menge an Olefinen mit 2n Kohlenstoffatomen. Ist die gemäß (b) gebildete Menge an Olefinen mit 2n Kohlenstoffatomen größer als die gemäß (a) verbrauchte Menge an Olefinen mit 2n Kohlenstoffatomen, liegt ein negativer Umsatz, d. h. eine Netto-Bildung an Olefinen mit 2n Kohlenstoffatomen, vor.

Das erfindungsgemäße Verfahren arbeitet mit einem Umsatz an Olefinen mit 2n Kohlenstoffatomen von kleiner 5%, insbesondere 0%.

Der Umsatz an Olefinen mit 2n Kohlenstoffatomen ist bei kontinuierlicher Verfahrensführung feststellbar durch einen Vergleich des Mengenstroms an Olefinen mit 2n Kohlenstoffatomen im Umsetzungsprodukt, der das Verfahren verlässt, mit dem Mengenstrom an Olefinen mit 2n Kohlenstoffatomen im Einsatzmaterial, der dem Verfahren zugeführt wird. Der Mengenstrom wird als Masse pro Zeiteinheit bzw. Stoffmenge pro Zeiteinheit angegeben. Der Umsatz ist auf den Mengenstrom an Olefinen mit 2n Kohlenstoffatomen im Einsatzmaterial bezogen, der dem Verfahren zugeführt wird. Erfindungsgemäß ist der das Verfahren verlassende Mengenstrom an Olefinen mit 2n Kohlenstoffatomen größer als 90% des zugeführten Mengenstroms an Olefinen mit 2n Kohlenstoffatomen.

Man kann das erfindungsgemäße Verfahren daher zweckmäßigerweise so regeln, dass man wenigstens eine Regelgröße, die den Netto-Umsatz an Olefinen mit 2n Kohlenstoffatomen beschreibt, misst und Stelleingriffe zur Regelung der Regelgröße ermittelt. Z. B. ist wenigstens eine Regelgröße die Menge an Olefinen mit 2n Kohlenstoffatomen im Umsetzungsprodukt. Die Bestimmung dieser Menge erfolgt entweder durch dem Fachmann bekannte analytische Methoden, z.B. Online-GC, oder durch Messung der Menge der entsprechenden Fraktion, die in der der Reaktion nachgeschalteten destillativen Auftrennung anfällt. Es können selbstverständlich weitere Regelgrößen gemessen werden. Als Stellgröße verwendet man z. B. wenigstens eine Größe, die ausgewählt ist unter der Verweilzeit des Olefin-Einsatzmaterials am Olefin-Oligomerisierungskatalysator, dem Mengenstrom des Olefin-Einsatzmaterials, dem Mengenstrom eines gegebenenfalls vorhandenen Rückführ- oder Wälzstroms, dem Verhältnis von Olefinen mit n Kohlenstoffatomen und Olefinen mit 2n Kohlenstoffatomen im Olefin-Einsatzmaterial und der Reaktionstemperatur am Reaktoreingang und Reaktorausgang.

Die Regelung kann durch eine computerbasierte Prozesssteuerung erfolgen. In einer Steuereinheit kann der Einfluss der Änderung einer Stellgröße auf eine oder mehrere Regelgrößen als mathematisches Modell bzw. Algorithmus hinterlegt werden. Aus den gemessenen Werten einer oder mehrerer Regelgrößen werden Stelleingriffe zur Regelung der Regelgröße ermittelt. Geeignete Modelle und Programme, die zur Implementierung der vorliegenden Erfindung Anwendung finden können, sind dem Fachmann geläufig. Im einfachsten Fall erfolgt die Regelung manuell, indem der Operateur bei einer Veränderung der Regelgröße entsprechende Stellgrößen anpasst.

Das molare Verhältnis von Olefinen mit n Kohlenstoffatomen zu Olefinen mit 2n Kohlenstoffatomen liegt in einem Bereich von 1 : 10 bis 20 : 1 vorzugsweise in einem Bereich von 1 : 4 bis 8 : 1, besonders bevorzugt in einem Bereich von 1 : 2 bis 4 : 1, insbesondere in einem Bereich von 1 : 1 bis 2,5 : 1.

Das erhaltene Umsetzungsprodukt kann in üblicher Weise, z. B. durch Destillation, aufgetrennt werden in eine nicht umgesetzte Olefine mit n Kohlenstoffatomen enthaltende Fraktion, Olefine mit 2n Kohlenstoffatomen, Olefine mit 3n Kohlenstoffatomen und gegebenenfalls höher siedende Fraktionen. Die Olefine mit 2n Kohlenstoffatomen und Olefine mit 3n Kohlenstoffatomen können verschiedenen Verwendungen zugeführt werden, z. B. einer Hydroformylierung.

In einer speziellen Ausführungsform wird das Umsetzungsprodukt in einen ersten und einen zweiten Teilstrom aufgetrennt, der erste Teilstrom einer Aufarbeitung unterzogen und der zweite Teilstrom zurückgeführt. Dieser zurückgeführte Teilstrom kann zuvor durch indirekten Wärmetausch gekühlt werden.

In einer speziellen Ausführung wird in das Reaktionssystem zusätzlich ein bei der Aufarbeitung des Umsetzungsprodukts bzw. des ersten Teilstroms des Umsetzungsprodukts gewonnener olefinhaltiger Strom eingespeist.

Vom Umsetzungsprodukt abgetrennte Olefine mit 3n Kohlenstoffatomen können gewünschtenfalls in eine weitere Co-Oligomerisierung zusammen mit Olefinen mit n Kohlenstoffatomen überführt werden, wobei man die weitere Co-Oligomerisierung unter solchen Bedingungen durchführt, dass der die weitere Co-Oligomerisierung verlassende Mengenstrom an Olefinen mit 3n Kohlenstoffatomen größer als 90% des zugeführten Mengenstroms an Olefinen mit 3n Kohlenstoffatomen ist.

Zur Vermeidung von Nebenreaktion und zur besseren Abfuhr der Reaktionswärme ist es bevorzugt, das Verfahren in mehreren Stufen, jeweils unter Teilumsatz der Olefine mit n Kohlenstoffatomen durchzuführen. Vorzugsweise führt man das Verfahren unter solchen Bedingungen durch, dass der Umsatz der Olefine mit n Kohlenstoffatomen in jeder einzelnen Stufe im Bereich von 5 bis 50 % liegt. Dabei ist als Umsatz definiert die Differenz der Masseströme aller olefinischen Kohlenwasserstoffe mit n Kohlenstoffatomen im Zulauf und im Ablauf des Reaktors geteilt durch den Massenstrom der olefinischen Kohlenwasserstoffe mit n Kohlenstoffatomen des Zulaufs.

Bevorzugte Olefine mit n Kohlenstoffatomen sind prinzipiell alle Verbindungen, welche 3 bis 10 Kohlenstoffatome, vorzugsweise 4 bis 6 Kohlenstoffatome, und wenigstens eine ethylenisch ungesättigte Doppelbindung aufweisen. Bevorzugt sind die eingesetzten Olefine ausgewählt unter linearen (geradkettigen) Olefinen und Olefingemischen, die wenigstens ein lineares Olefin enthalten. Dazu zählen Propen, 1-Buten, 2-Buten, 1-Penten, 2-Penten, 1-Hexen, 2-Hexen, 3-Hexen und Mischungen davon.

Vorzugsweise wird ein technisch zur Verfügung stehendes olefinhaltiges Kohlenwasserstoffgemisch als Olefin-Einsatzmaterial mit n Kohlenstoffatomen eingesetzt. Dabei kann das monomere Olefin-Einsatzmaterial im Allgemeinen neben Olefinen auch gesättigte Kohlenwasserstoffe, überwiegend mit der jeweils gleichen Kohlenstoffzahl, umfassen. Solche Olefin-Einsatzströme treten häufig als Cracker-Produkte auf, z. B. als so genannte C4 oder C5-Schnitte bzw. den daraus gewonnenen Raffinaten.

Bevorzugte Quelle für Olefine mit n Kohlenstoffatomen sind großtechnisch zur Verfügung stehende Olefingemische, die aus der Kohlenwasserstoff-Spaltung bei der Erdölverarbeitung, beispielsweise durch Kat-cracken, wie Fluid Catalytic Cracking (FCC), Thermocracken oder Hydrocracken mit anschließender Dehydrierung resultieren. Ein geeignetes technisches Olefingemisch ist ein C₄-Schnitt. C₄-Schnitte sind beispielsweise durch Fluid Catalytic Cracking oder Steamcracken von Gasöl bzw. durch Steamcracken von Naphtha erhältlich. Je nach Zusammensetzung des C₄-Schnitts unterscheidet man den Gesamt-C₄-Schnitt (Roh-C₄-Schnitt), das nach der Abtrennung von 1,3-Butadien erhaltene so genannte Raffinat I sowie das nach der Isobutenabtrennung erhaltene Raffinat II. Ein weiteres geeignetes technisches erstes Olefingemisch ist der bei der Naphtha-Spaltung erhältliche C₅-Schnitt. Geeignete olefinhaltige Kohlenwasserstoffgemische mit 4 bis 6 Kohlenstoffatomen lassen sich weiterhin durch katalytische Dehydrierung geeigneter großtechnisch zur Verfügung stehender Paraffingemische erhalten. So gelingt beispielsweise die Herstellung von C₄-Olefin-Gemischen aus Flüssiggasen (liquified petroleum gas, LPG) und verflüssigbaren Erdgasen (liquified natural gas, LNG). Letztere umfassen neben der LPG-Fraktion auch zusätzlich größere Mengen höhermolekularer Kohlenwasserstoffe (leichtes Naphtha) und eignen sich somit auch zur Herstellung von C₅- und C₆-Olefin-Gemischen. Die Herstellung von olefinhaltigen Kohlenwasserstoffgemischen, die Monoolefine mit 4 bis 6 Kohlenstoffatomen enthalten, aus LPG- oder LNG-Strömen gelingt nach üblichen, dem Fachmann bekannten Verfahren, die neben der Dehydrierung in der Regel noch einen oder mehrere Aufarbeitungsschritte umfassen. Dazu zählt beispielsweise die Abtrennung wenigstens eines Teils der in den zuvor genannten Olefin-Einsatzgemischen enthaltenen gesättigten Kohlenwasserstoffe. Diese können beispielsweise erneut zur Herstellung von Olefin-Einsatzmaterialien durch Crackung und/oder Dehydrierung eingesetzt werden. Die in dem erfindungsgemäßen Verfahren eingesetzten Olefine können jedoch auch einen Anteil gesättigter Kohlenwasserstoffe enthalten, die sich gegenüber den erfindungsgemäßen Oligomerisierungsbedingungen inert verhalten. Der Anteil dieser gesättigten Komponenten beträgt im Allgemeinen höchstens 60 Gew.-%, bevorzugt höchstens 40 Gew.-%, besonders bevorzugt höchstens 30 Gew.-%, bezogen auf die Gesamtmenge der in dem Kohlenwasserstoff-Einsatzmaterial enthaltenen Olefine und gesättigten Kohlenwasserstoffe.

Ein zum Einsatz in dem erfindungsgemäßen Verfahren geeignetes Raffinat II hat beispielsweise die folgende Zusammensetzung:
0,5 bis 5 Gew.-% Isobutan,
5 bis 30 Gew.-% n-Butan,
20 bis 40 Gew.-% trans-2-Buten,
10 bis 20 Gew.-% cis-2-Buten,
25 bis 55 Gew.-% 1-Buten,
0,5 bis 5 Gew.-% Isobuten
sowie Spurengase, wie 1,3-Butadien, Propen, Propan, Cyclopropan, Propadien, Methylcyclopropan, Vinylacetylen, Pentene, Pentane etc. im Bereich von jeweils maximal 1 Gew.-%.

Sind Diolefine oder Alkine im olefinreichen Kohlenwasserstoffgemisch vorhanden, so können diese vor der Oligomerisierung auf vorzugsweise weniger als 200 Gew.-ppm aus demselben entfernt werden. Sie werden bevorzugt durch selektive Hydrierung, z. B. gemäß EP-81 041 und DE-15 68 542 entfernt, besonders bevorzugt durch eine selektive Hydrierung bis auf einen Restgehalt von unter 100 Gew.-ppm, insbesondere 10 Gew.-ppm.

Aus dem olefinreichen Kohlenwasserstoffgemisch werden zweckmäßigerweise außerdem sauerstoffhaltige Verbindungen, wie Alkohole, Aldehyde, Ketone oder Ether, weitgehend entfernt. Hierzu kann das olefinreiche Kohlenwasserstoffgemisch mit Vorteil über ein Adsorptionsmittel, wie z. B. ein Molekularsieb, bevorzugt ein Adsorptionsmittel wie in der DE-A-19845857 beschrieben, worauf diesbezüglich Bezug genommen wird, geleitet werden. Die Konzentration an sauerstoffhaltigen, schwefelhaltigen, stickstoffhaltigen und halogenhaltigen Verbindungen im olefinreichen Kohlenwasserstoffgemisch beträgt vorzugsweise weniger als 20 Gew.-ppm, besonders bevorzugt weniger als 10 Gew.-ppm, insbesondere weniger als 1 Gew.-ppm.

Olefine mit 2n Kohlenstoffatomen sind vorzugsweise solche, die durch vorgelagerte Dimerisierung von Olefinen mit n Kohlenstoffatomen erhalten sind. Olefine mit 2n Kohlenstoffatomen sind insbesondere Olefine mit 8 Kohlenstoffatomen, d. h. Octene. Bevorzugt sind die zur Oligomerisierung eingesetzten Olefine ausgewählt unter linearen und wenig verzweigten Olefinen und Olefingemischen. Auch das C₂ₙ-Olefingemisch kann vor dem Eintrag in den Co-Oligomerisierungsreaktor einer geeigneten Reinigung zur Entfernung sauerstoff-, schwefel- oder stickstoffhaltiger Verbindungen sowie konjugiert mehrfach ungesättigter Olefine unterzogen werden. C₂ₙ-Olefingemische können herstellungsbedingt geringe Mengen gelösten Sauerstoffs enthalten, auch dieser kann zum Schutz des Oligomerisierungskatalysators durch geeignete dem Fachmann bekannte adsorptive oder chemische, insbesondere katalytische, Maßnahmen entfernt werden.

Geeignete Octene sind z. B. 1-Octen, 2-Octen, 3-Octen, 4-Octen, 2-Methyl-hept-1-en, 2-Methyl-hept-2-en, 2-Methyl-hept-3-en, 6-Methyl-hept-3-en, 6-Methyl-hept-2-en, 6-Methyl-hept-1-en, 3-Methyl-hept-1-en, 3-Methyl-hept-2-en, 3-Methyl-hept-3-en, 5-Methyl-hept-3-en, 5-Methyl-hept-2-en, 5-Methyl-hept-1-en, 4-Methyl-hept-1-en, 4-Methyl-hept-2-en, 4-Methyl-hept-3-en, und Mischungen davon.

Bevorzugte großtechnisch zur Verfügung stehende C₈-Olefingemische resultieren beispielsweise beim DIMERSOL-Prozess, bei dem Buten in homogener Phase in Gegenwart eines Katalysatorsystems aus einem Übergangsmetallderivat und einer metallorganischen Verbindung oligomerisiert wird (Revue de l'Institut Francais du Petrole, Vol. 37, No. 5, Sept./Okt. 1982, S. 639ff). Als zweites Olefin-Einsatzmaterial geeignete C₈-Olefingemische resultieren auch aus dem Octol-Verfahren der Firma Hüls AG (Hydrocarbon Processing, Februar 1992, S. 45/46). Geeignete Verfahren zur Herstellung von wenig verzweigten C₈-Olefingemischen sind weiterhin in der DE-A-43 39 713 und WO 99/25668 beschrieben, auf die hier in vollem Umfang Bezug genommen wird. In einer bevorzugten Ausführung werden Olefine mit 2n Kohlenstoffatomen durch Dimerisierung eines Raffinats II, wie zuvor definiert, in Gegenwart eines Nickel-haltigen Oligomerisierungskatalysators erhalten.

Eine besonders bevorzugte Ausführungsform betrifft ein Verfahren, bei dem man
(i) in einer Dimerisierungsstufe ein Olefin-Einsatzmaterial, das Olefine mit n Kohlenstoffatomen umfasst, an einem ersten Olefin-Oligomerisierungskatalysator zu einem ersten Umsetzungsprodukt umsetzt,
   aus dem ersten Umsetzungsprodukt Olefine mit 2n Kohlenstoffatomen isoliert, und
(ii) in einer Co-Oligomerisierungsstufe ein Olefin-Einsatzmaterial, das Olefine mit n Kohlenstoffatomen und zumindest einen Teil der in der Dimerisierungsstufe gewonnenen Olefine mit 2n Kohlenstoffatomen umfasst, an einem zweiten Olefin-Oligomerisierungskatalysator zu einem zweiten Umsetzungsprodukt umsetzt, wobei man die Co-Oligomerisierung unter solchen Bedingungen durchführt, dass der Umsatz an Olefinen mit 2n Kohlenstoffatomen kleiner 10% ist.

Vorzugsweise ist zumindest der zweite Olefin-Oligomerisierungskatalysator ein Nickel enthaltender heterogener Katalysator; insbesondere sind der erste Olefin-Oligomerisierungskatalysator und der zweite Olefin-Oligomerisierungskatalysator jeweils ein Nickel enthaltender heterogener Katalysator, wie nachstehend erläutert.

Die Co-Oligomerisierung erfolgt vorzugsweise kontinuierlich. Dazu wird in ein Reaktorsystem das Olefin-Einsatzmaterial, das Olefine mit n Kohlenstoffatomen und Olefine mit 2n Kohlenstoffatomen umfasst, eingespeist und an dem Olefin-Oligomerisierungskatalysator umgesetzt.

Das im erfindungsgemäßen Verfahren eingesetzte Reaktionssystem kann einen oder mehrere, gleiche oder verschiedene Reaktoren umfassen. Im einfachsten Fall wird das Reaktionssystem von einem einzelnen Reaktor gebildet. Werden mehrere Reaktoren eingesetzt, so können diese jeweils gleiche oder verschiedene Vermischungscharakteristiken aufweisen. Die einzelnen Reaktoren können gewünschtenfalls durch Einbauten ein- oder mehrfach unterteilt sein. Bilden zwei oder mehrere Reaktoren das Reaktionssystem, so können diese untereinander beliebig verschaltet sein, z. B. parallel oder in Reihe. In einer bevorzugten Ausführung wird ein Reaktionssystem eingesetzt, das aus zwei in Reihe geschalteten Reaktoren besteht.

Geeignete druckfeste Reaktionsapparaturen für die Oligomerisierung sind dem Fachmann bekannt. Dazu zählen die allgemein üblichen Reaktoren für Gas-fest- und Gasflüssig-Reaktionen, wie z. B. Rohrreaktoren, Rührkessel, Gasümlaufreaktoren, Blasensäulen etc., die gegebenenfalls durch Einbauten unterteilt sein können. Vorzugsweise werden Rohrbündelreaktoren oder Schachtöfen eingesetzt, die in Sumpf- oder Rieselfahrweise betrieben werden. In dem Reaktor oder den Reaktoren kann der Katalysator in einem einzigen oder in mehreren Katalysator-Festbetten angeordnet sein. Dabei ist es möglich, in den einzelnen Reaktionszonen unterschiedliche Katalysatoren einzusetzen. Bevorzugt ist jedoch der Einsatz des gleichen Katalysators in allen Reaktionszonen.

Die Temperatur bei der Co-Oligomerisierungsreaktion liegt im Allgemeinen in einem Bereich von etwa 20 bis 280 °C, bevorzugt von 25 bis 200 °C, insbesondere von 30 bis 140 °C. Umfasst das Reaktionssystem mehr als einen Reaktor, so können diese gleiche oder verschiedene Temperaturen aufweisen. Des Gleichen kann ein Reaktor mehrere Reaktionszonen aufweisen, die bei verschiedenen Temperaturen betrieben werden. So kann beispielsweise in einer zweiten Reaktionszone eines einzelnen Reaktors eine höhere Temperatur als in der ersten Reaktionszone oder im zweiten Reaktor einer Reaktorkaskade eine höhere Temperatur als im ersten Reaktor eingestellt werden, z. B. um einen möglichst vollständigen Umsatz zu erzielen.

Der Druck bei der Oligomerisierung liegt im Allgemeinen in einem Bereich von etwa 1 bis 300 bar, vorzugsweise von 5 bis 100 bar und insbesondere von 10 bis 70 bar. Der Reaktionsdruck kann beim Einsatz mehrerer Reaktoren in den einzelnen Reaktoren unterschiedlich sein.

Im Allgemeinen werden die zur Oligomerisierung eingesetzten Temperatur und Druckwerte so gewählt, dass das olefinhaltige Einsatzmaterial flüssig oder im überkritischen Zustand vorliegt.

Im Allgemeinen können die im Reaktionsgemisch enthaltenen olefinischen Komponenten unter den Reaktionsbedingungen neben den Oligomerisierungsreaktionen auch Isomerisierungsreaktionen eingehen. Diese Isomerisierungen betreffen überwiegend die Verschiebung der ethylenischen Doppelbindung entlang der Kohlenstoffkette, es können aber auch Skelettisomerisierungen, die zu einer Umlagerung der Kohlenstoffkette führen, auftreten. Insbesondere die Doppelbindungsisomerisierungen verlaufen exotherm.

Auch die Oligomerisierungsreaktion verläuft exotherm. Die Umsetzung kann adiabat oder unter Abführung der Reaktionswärme durch indirekten Wärmetausch mit einem externen Wärmeträgermedium erfolgen. Geeignete Vorrichtungen zum Wärmetausch und zur Abführung von Prozesswärme sind die üblichen, dem Fachmann bekannten. Die Wärmeaustauschvorrichtung kann an bzw. im Reaktor angebracht sein.

Vorzugsweise wird die Umsetzung adiabat durchgeführt. Dieser Begriff wird im Rahmen der vorliegenden Erfindung im technischen und nicht im physiko-chemischen Sinne verstanden. Das Reaktionsgemisch erfährt beim Strömen durch das Reaktionssystem, beispielsweise ein Katalysatorbett, eine Temperaturerhöhung. Unter adiabater Reaktionsführung wird eine Vorgehensweise verstanden, bei der die in einer exothermen Reaktion freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem Reaktor abgeführt, abgesehen von einem Restanteil, der durch natürliche Wärmeleitung und Wärmeabstrahlung vom Reaktor an die Umgebung abgegeben wird. Bei solcherart adiabater Betriebsweise stellt sich in dem jeweiligen Reaktor ein kontinuierliches Temperaturprofil in Strömungsrichtung ein.

Nach der zuvor beschriebenen Verfahrensvariante mit Rückführung eines Teilstroms des Umsetzungsprodukts kann dem Teilstrom Wärme durch indirekten Wärmetausch entzogen werden. Die gewonnene Wärmemenge kann an anderer Stelle des Verfahrens, z. B. bei der Auftrennung des Umsetzungsprodukts, wieder eingesetzt werden.

Bevorzugt handelt es sich bei dem Olefin-Oligomerisierungskatalysator um einen übergangsmetallhaltigen Katalysator, insbesondere einen heterogenen Katalysator. Geeignete Katalysatoren sind dem Fachmann bekannt. Dazu zählen die in Catalysis Today, 6, 329 (1990), insbesondere Seiten 336 - 338, sowie die in der DE-A-43 39 713 (= WO-A 95/14647) und der DE-A-199 57 173 beschriebenen Katalysatoren

Bevorzugt wird ein Oligomerisierungskatalysator eingesetzt, der Nickel enthält. Die eingesetzten heterogenen Nickel enthaltenden Katalysatoren können unterschiedliche Strukturen aufweisen. Geeignet sind prinzipiell Vollkatalysatoren sowie geträgerte Katalysatoren. Erstere werden bevorzugt eingesetzt. Die Trägermaterialien können z. B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z. B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von Ni²⁺-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Nickeloxid enthaltende Katalysatoren sind bevorzugt. Besonders bevorzugt sind Katalysatoren, die im Wesentlichen aus NiO, SiO₂, TiO₂ und/oder ZrO₂ sowie gegebenenfalls Al₂O₃ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE-43 39 713 verwiesen. Auf die Offenbarung dieser Druckschrift und den darin zitierten Stand der Technik wird vollinhaltlich Bezug genommen.

In einer weiteren Ausführungsform wird als Katalysator ein Nickelkatalysator gemäß der DE-A-199 57 173 eingesetzt. Dabei handelt es sich im Wesentlichen um Aluminiumoxid, das mit einer Nickelverbindung und einer Schwefelverbindung beaufschlagt wurde. Vorzugsweise liegt im fertigen Katalysator ein molares Verhältnis von Schwefel zu Nickel im Bereich von 0,25 : 1 bis 0,38 : 1 vor.

Der Katalysator liegt vorzugsweise in stückiger Form, z. B. in Form von Tabletten, z. B. mit einem Durchmesser von 1,5 bis 6 mm und einer Höhe von 1,5 bis 6 mm, Ringen mit z. B. 5 bis 7 mm Außendurchmesser, 2 bis 5 mm Höhe und 2 bis 3 mm Lochdurchmesser, oder Strängen unterschiedlicher Länge eines Durchmessers von z. B. 1,5 bis 5 mm, vor. Derartige Formen werden auf an sich bekannte Weise durch Tablettierung, meist unter Verwendung eines Tablettierhilfsmittels, wie Graphit oder Stearinsäure oder Extrusion erhalten.

In weniger bevorzugten Ausführungsformen umfasst der Olefin-Oligomerisierungskatalysator wenigstens einen Zeolith oder besteht aus wenigstens einem Zeolith.

Geeignete Zeolithe weisen einen mittleren Porendurchmesser von wenigstens 5 Å, besonders bevorzugt wenigstens 6 Å insbesondere wenigstens 7 Å auf.

Geeignete Zeolithe sind ausgewählt unter folgenden Strukturtypen (Bezeichnung folgt der Nomenklatur der International Zeolite Association): BEA, MFI, MEL, FAU, MOR, MWW, LTL, LTA, CHA, TON, MTW, FER, MAZ, EPI und GME.

Die eingesetzten Zeolithe, können z. B. in der H⁺, Ammonium-, Alkali- oder Erdalkali-Form eingesetzt werden.

Die eingesetzten Zeolithe, können vor ihrem Einsatz zur Olefin-Oligomerisierung wenigstens einem Modifizierungsschritt unterzogen werden. Dazu zählt z. B. eine Modifizierung mit Säuren, Ammonium- und/oder mit Metallsalzlösungen. Dazu zählt weiterhin eine Dealuminierung des in des Silicatgerüsts eingebauten Aluminiums, Dehydroxylierung, Extraktion von "extra-framework" Aluminiumoxid oder Silylierung. Des Weiteren kann der Olefin-Oligomerisierungskatalysator zur Modifizierung einer Formgebung, thermischen Behandlung oder einer Behandlung mit Wasserdampf (steaming) unterzogen werden. Durch eine solche Modifizierung ist es möglich, eine möglichst hohe Selektivität, hohe Umsätze, lange Katalysator-Standzeiten und/oder eine hohe Anzahl möglicher Regenerationszyklen zu erreichen.

In einer Ausführungsform des erfindungsgemäßen Verfahrens wird als Olefin-Oligomerisierungskatalysator ein Zeolith in der H⁺-Form eingesetzt.

Die Erfindung wird durch die folgenden Beispiele näher veranschaulicht.

### Vergleichsbeispiele 1 und 3 und Beispiel 2:

In den Beispielen 1 und 2 wird Buten (Raffinat II folgender Zusammensetzung: 7,1 Gew.% Isobutan, 19,3 Gew.% n-Butan, 19,9 Gew.% trans-2-Buten, 41,6 Gew.% 1-Buten, 9,5 Gew. Cis-2-Buten und 2,6 Gew.% Isobuten) mit Octen, welches zuvor in einer ersten Reaktionseinheit durch Oligomerisation von Raffinat II an einem NiObasierten Katalysator (EP 730567 B Beispiel 1) gewonnen wurde, an einem zeolithischen sauren Katalysator basierend auf einem H-MWW-Zeolith (molares Verhäftnis der Element Si:Al:Fe 27:1:0,07, verstrangt mit 20% Böhmit als Binder) zu Dodecen oligomerisiert. Das molare Verhältnis C₄:C₈ betrug jeweils 2 :1. Die Reaktion erfolgte in einem kontinuierlich betriebenen Reaktor mit einem Durchmesser von 29,7 mm und einer Gesamtlänge von 3 m bei 90°C und dem in der nachstehenden Tabelle angegebenen stündlichen Massendurchsatz (weight hourly space velocity WHSV).

In Beispiel 3 erfolgt die gleiche Reaktion mit einem molaren Verhältnis von C4- zu C8-Olefinen von 1 : 1 im Eingangsstrom an einem NiO basierten Katalysator, wie er bereits für die Herstellung des Octens eingesetzt wurde.

Das gebildete Produkt wird jeweils destillativ aufgetrennt und das so erhaltene Dodecen hydroformyliert. Hierzu wird ein Autoklav mit 1200 g Dodecen befüllt. Der Autoklav wird sodann mit einem CO/H₂-Gemisch (1:1) auf 220 bar aufgepresst und auf 185°C geheizt. Dann werden 9,6 g Cobaltethylhexanoat gelöst in ca. 100 g Dodecen in den Reaktor mittels einer Schleuse eingebracht. Der Verbrauch an Synthesegas, zu erkennen an einem Druckabfall im Autoklaven wurde durch Nachpressen ergänzt. Zur Bestimmung der Hydroformylierbarkeit wird nach 60 min eine Probe entnommen und gaschromatographisch analysiert.

| | Co-Oligomerisierung | | | | | |
|---|---|---|---|---|---|---|
| Beispiel | Katalysator | WHSV | Temperatur | Umsatz C4 | Umsatz C8 | Hydroformylierumsatz |
| | | 1/h | °C | % | % | % |
| 1 | H-MWW | 0,4 | 90 | 54 | 11 | 55 |
| 2 | H-MWW | 1,6 | 90 | 35 | 3 | 61 |
| 3 | NiO | 1,1 | 90 | 38 | -4 | 81 |

Es zeigt sich, dass bei Verwendung der gleichen Katalysatoren die Hydroformylierbarkeit des Dodecens mit fallendem C8-Umsatz deutlich ansteigt. Desweiteren zeigt sich, dass bei der bevorzugten Verwendung gleicher Katalysatoren in der C4-Dimerisierung und in der Co-Dimerisierung von C4- und C8-Olefinen bei niedrigem C8-Umsatz (hier <0%) besonders gut hydroformylierbares Dodecen erhältlich ist

### Vergleichsbeispiele 4 und 5 und Beispiele 6 bis 8

In den nachfolgenden Beispielen wird ein Eingangsgemisch aus C4- und C8-Olefinen, entsprechend dem Beispiel 3 an einem NiO basierten Katalysator (entsprechend EP 730567 B Beispiel 1) bei 95°C und verschiedenen Durchflussmengen umgesetzt. Von jeder einstellung wurden Proben entnommen und zur Trennung der C8- und C12-Olefine destillativ aufgearbeitet. Die so rein erhaltenen C8- und C12-Olefinfraktionen wurden jeweils einer Hydroformylierung wie folgt unterzogen:

100 g Dodecen (Octen) wurden diskontinuierlich in einem Autoklav mit 0,13 Gew.-% Co-Ethylhexaoate als Katalysator unter Zusatz von 10 g Wasser bei 175 °C (160°C) und unter einem Synthesegasdruck von 280 bar bei einem Mischungsverhältnis von CO zu H₂ von 1:1 4 Stunden umgesetzt. Der Verbrauch an Synthesegas, zu erkennen an einem Druckabfall in Autoklaven wurde durch Nachpressen ergänzt. Nach dem entspannen des Autoklaven wurde der Reaktionsaustrag mit 10 Gew.%iger Essigsäure durch Einleiten von Luft oxidativ von Cobaltkatalysator befreit und die organische Produktphase mit Raney-Nickel bei 170 °C und einem Wasserstoffdruck von 280 bar hydriert. Die Analyse des erhalten Produktgemischs erfolgte gaschromatographisch.

| | Co-Oligomerisierung | | | | | |
|---|---|---|---|---|---|---|
| Beispiel | WHSV | Umsatz C4 | Umsatz C8 | Hydroformylierumsatz C8 | Hydroformylierumsatz C12 | Mittlerer Hydroformylierumsatz |
| | 1/h | % | % | % | % | % |
| 4 | 0,15 | 43 | 17 | 97,9 | 79,5 | 88,7 |
| 5 | 0,37 | 37 | 15 | 95,9 | 80,1 | 88,0 |
| 6 | 0,67 | 28 | 4 | 95,9 | 85,7 | 90,8 |
| 7 | 1,34 | 23 | 1 | 98,3 | 88,4 | 93,4 |
| 8 | 2,24 | 21 | 0 | 98,1 | 86,3 | 92,2 |

Es zeigt sich hierbei, dass bei hohem C8-Umsatz zwar ein gut hydroformylierbares Octen aber kein gut hydroformylierbares Dodecen erhalten wird. Erst bei niedrigen C8-Umsätzen kleiner 10% sind beide Produkte gut hydroformylierbar, was durch Mittelwertbildung über die Hydroformylierumsätze des C8- und C12-Olefins deutlich wird.

## Patentansprüche

1. Verfahren zur Co-Oligomerisierung von Olefinen, bei dem man ein Olefin-Einsatzmaterial, das Olefine mit n Kohlenstoffatomen und Olefine mit 2n Kohlenstoffatomen umfasst, an einem Olefin-Oligomerisierungskatalysator zu einem Umsetzungsprodukt umsetzt, wobei n für eine ganze Zahl von 3 bis 10 steht und man das Verfahren unter solchen Bedingungen durchführt, dass der Umsatz an Olefine mit 2n Kohlenstoffatomen nicht-negativ und kleiner als 5% ist und der Umsatz der Olefine mit n Kohlenstoffatomen im Bereich von 10 bis 50 % liegt.

2. Verfahren nach Anspruch 1, wobei der Umsatz an Olefinen mit 2n Kohlenstoffatomen kleiner oder gleich 1% ist.

3. Verfahren nach Anspruch 1 oder 2, wobei man eine Teilmenge des Umsetzungsprodukts durch indirekten Wärmetausch kühlt und zum Olefin-Einsatzmaterial zurückführt.

4. Verfahren nach einem der vorhergehenden Ansprüche, wobei man als Olefin-Oligomerisierungskatalysator einen Nickel enthaltenden heterogenen Katalysator verwendet.

5. Verfahren nach einem der Ansprüche 1 bis 3, wobei man als Olefin-Oligomerisierungskatalysator einen zeolithischen heterogenen Katalysator verwendet.

6. Verfahren nach einem der vorhergehenden Ansprüche, wobei man die Menge an Olefinen mit 2n Kohlenstoffatomen im erhaltenen Umsetzungsprodukt durch geeignete Einstellung wenigstens einer Stellgröße regelt.

7. Verfahren nach Anspruch 6, wobei man als Stellgröße wenigstens eine Größe verwendet, die ausgewählt ist unter der Verweilzeit des Olefin-Einsatzmaterials am Olefin-Oligomerisierungskatalysator, dem Mengenstrom des Olefin-Einsatzmaterials, dem Mengenstrom eines Rückführ- oder Wälzstroms, dem Verhältnis von Olefinen mit n Kohlenstoffatomen und Olefinen mit 2n Kohlenstoffatomen im Olefin-Einsatzmaterial und der Reaktionstemperatur.

8. Verfahren nach einem der vorhergehenden Ansprüche, umfassend eine vorgelagerte Dimerisierungsstufe, bei der man ein Olefin-Einsatzmaterial, das Olefine mit n Kohlenstoffatomen umfasst, an einem Olefin-Oligomerisierungskatalysator zu Olefinen mit 2n Kohlenstoffatomen umsetzt.

9. Verfahren nach einem der vorhergehenden Ansprüche, wobei man als Olefine mit n Kohlenstoffatomen im Wesentlichen n-Butene und als Olefine mit 2n Kohlenstoffatomen Isooctene einsetzt.

## Claims

1. A process for the cooligomerization of olefins, wherein an olefin starting material comprising olefins having n carbon atoms and olefins having 2n carbon atoms is reacted over an olefin oligomerization catalyst to give a reaction product, n is an integer from 3 to 10 and the process is carried out under such conditions that the conversion of olefins having 2n carbon atoms is non-negative and less than 5% and the conversion of olefins having n carbon atoms is in the range from 10 to 50%.

2. The process according to claim 1, wherein the conversion of olefins having 2n carbon atoms is less than or equal to 1%.

3. The process according to claim 1 or 2, wherein a part of the reaction product is cooled by indirect heat exchange and recirculated to the olefin starting material.

4. The process according to any of the preceding claims, wherein a nickel-comprising heterogeneous catalyst is used as olefin oligomerization catalyst.

5. The process according to any of claims 1 to 3, wherein a zeolitic heterogeneous catalyst is used as olefin oligomerization catalyst.

6. The process according to any of the preceding claims, wherein the amount of olefins having 2n carbon atoms in the reaction product obtained is regulated by targeted setting of at least one correcting variable.

7. The process according to claim 6, wherein at least one parameter selected from the residence time of the olefin starting material over the olefin oligomerization catalyst, the mass flow of the olefin starting material, the mass flow of a recycle or circulation stream, the ratio of olefins having n carbon atoms to olefins having 2n carbon atoms in the olefin starting material and the reaction temperature is used as correcting variable.

8. The process according to any of the preceding claims, which comprises an upstream dimerization stage in which an olefin starting material comprising olefins having n carbon atoms is reacted over an olefin oligomerization catalyst to form olefins having 2n carbon atoms.

9. The process according to any of the preceding claims, wherein essentially n-butenes are used as olefins having n carbon atoms and isooctenes are used as olefins having 2n carbon atoms.

## Revendications

1. Procédé pour la co-oligomérisation d'oléfines, dans lequel on convertit un produit de départ comportant des oléfines, qui comporte des oléfines ayant n atomes de carbone et des oléfines ayant 2n atomes de carbone, sur un catalyseur d'oligomérisation d'oléfines, en un produit de conversion, n représentant un nombre entier valant de 3 à 10 et on effectue le procédé dans des conditions telles que le taux de conversion des oléfines ayant 2n atomes de carbone est non négatif et inférieur à 5 % et le taux de conversion des oléfines ayant n atomes de carbone se situe dans la plage de 10 à 50 %.

2. Procédé selon la revendication 1, dans lequel le taux de conversion des oléfines ayant 2n atomes de carbone est inférieur ou égal à 1 %.

3. Procédé selon la revendication 1 ou 2, dans lequel on refroidit par échange thermique indirect une quantité partielle du produit de conversion et on la renvoie au produit de départ comportant des oléfines.

4. Procédé selon l'une quelconque des revendications précédentes, dans lequel on utilise comme catalyseur d'oligomérisation d'oléfines un catalyseur hétérogène contenant du nickel.

5. Procédé selon l'une quelconque des revendications 1 à 3, dans lequel on utilise comme catalyseur d'oligomérisation d'oléfines un catalyseur hétérogène de type zéolithe.

6. Procédé selon l'une quelconque des revendications précédentes, dans lequel on règle la quantité d'oléfines comportant 2n atomes de carbone, dans le produit de conversion obtenu, par ajustement convenable d'au moins une grandeur de commande.

7. Procédé selon la revendication 6, dans lequel on utilise au moins une grandeur qui est choisie parmi le temps de séjour du produit de départ comportant des oléfines sur le catalyseur d'oligomérisation d'oléfines, le flux massique du produit de départ comportant des oléfines, le flux massique d'un courant de recyclage ou de circulation, le rapport d'oléfines ayant n atomes de carbone et d'oléfines ayant 2n atomes de carbone dans le produit de départ comportant des oléfines et la température de réaction.

8. Procédé selon l'une quelconque des revendications précédentes, comprenant une étape préliminaire de dimérisation, dans laquelle on convertit un produit de départ comportant des oléfines, qui comprend des oléfines ayant n atomes de carbone, sur un catalyseur d'oligomérisation d'oléfines, en oléfines ayant 2n atomes de carbone.

9. Procédé selon l'une quelconque des revendications précédentes, dans lequel on utilise comme oléfines ayant n atomes de carbone essentiellement des n-butènes et comme oléfines ayant 2n atomes de carbone des iso-octènes.
